# FASCICULE DE BREVET EUROPEEN

(11) **EP 2 167 102 B1**
(45) Date de publication et mention de la délivrance du brevet: **25.05.2016**
(21) Numéro de dépôt: 08827623.3
(22) Date de dépôt: 04.07.2008
(51) Int. Cl.: A61K 31/728, A61K 45/06, A61P 1/04, A61P 17/00, A61P 27/02, A61P 27/04, A61P 11/00

(54) **UTILISATION D'ACIDE HYALURONIQUE POUR LA PREPARATION DE COMPOSITIONS DESTINEES A L'AMELIORATION DE LA FONCTION DE PROTECTION DE LA PEAU, DE L'OEIL ET DES MUQUEUSES**
VERWENDUNG VON HYALURONSÄURE ZUR HERSTELLUNG VON ZUSAMMENSETZUNGEN INSBESONDERE ZUR VERBESSERUNG DER FUNKTION DER HAUT UND DER AUGEN SOWIE ZUM SCHUTZ DER MUKOSEMEMBRAN
USE OF HYALURONIC ACID FOR PREPARING COMPOSITIONS PARTICULARLY FOR IMPROVING THE FUNCTION OF SKIN, EYE AND MUCOUS MEMBRANE PROTECTION

(30) Priorité: 23.07.2007 FR 0705349
(43) Date de publication de la demande: 31.03.2010
(73) Titulaire: Agro Industrie Recherches et Developpements (A.R.D.), 51110 Pomacle (FR); INSERM (Institut National de la Santé et de la Recherche Médicale), 75654 Paris Cedex 13 (FR)
(72) Inventeur: BRESIN, Anthony, F-54490 Saint Masmes (FR); PUCHELLE, Edith, F-33710 Tauriac (FR); ZAHM, Jean-Marie, F-51100 Reims (FR); MILLIOT, Magali, F-51100 Reims (FR)
(74) Mandataire: Grosset-Fournier, Chantal Catherine
(86) Numéro de dépôt international: PCT/FR2008/000959
(87) Numéro de publication internationale: WO 2009/024677

(56) Documents cités:
- WO-A-00/01394
- WO-A-02/069984
- WO-A-2006/072243
- FR-A- 2 847 818
- US-A- 5 166 331
- NAKAMURA M ET AL: "Recent developments in the use of hyaluronan in wound healing" EXPERT OPINION ON INVESTIGATIONAL DRUGS, ASHLEY PUBLICATIONS LTD., LONDON, GB, vol. 4, no. 3, 1 janvier 1995 (1995-01-01), pages 175-188, XP008097254 ISSN: 1354-3784
- "Martindale" 1999, PHARMACEUTICAL PRESS , LONDON, UK 32 , XP002548563 page 1365 - page 1366 page 1369 - page 1370
- MORGANTI P ET AL: "Biweekly in-office injectable treatment of striae distensae vs a long-term daily use of topical vitamin C" JOURNAL OF APPLIED COSMETOLOGY 200110 IT, vol. 19, no. 4, octobre 2001 (2001-10), pages 107-112, XP008112941 ISSN: 0392-8543
- DI PIETRO A ET AL: "Role of hyaluronic acid and vitamin C in photoageing" JOURNAL OF APPLIED COSMETOLOGY 1998 IT, vol. 16, no. 4, 1998, pages 125-133, XP008112939 ISSN: 0392-8543
- HILL DAVID R ET AL: "Specific-sized Hyaluronan Fragments Promote Expression of Human beta-Defensin 2 in Intestinal Epithelium", JOURNAL OF BIOLOGICAL CHEMISTRY, vol. 287, no. 36, August 2012 (2012-08), pages 30610-30624, ISSN: 0021-9258(print)
- BAUMANN L: "Skin ageing and its treatment.", THE JOURNAL OF PATHOLOGY JAN 2007, vol. 211, no. 2, January 2007 (2007-01), pages 241-251, ISSN: 0022-3417
- YOKOI NORIHIKO ET AL: "Effectiveness of hyaluronan on corneal epithelial barrier function in dry eye", BRITISH JOURNAL OF OPHTHALMOLOGY, vol. 81, no. 7, 1997, pages 533-536, ISSN: 0007-1161

## Description

La présente invention concerne l'utilisation d'acide hyaluronique pour la préparation de compositions destinées à l'amélioration de la fonction de protection de l'oeil et des muqueuses, en particulier des voies aériennes supérieures et inférieures et de la muqueuse intestinale.

La muqueuse des voies aériennes et la muqueuse intestinale, la peau et l'oeil sont tapissées d'un épithélium de surface formant un tapis continu et constituant une barrière physique de protection dont l'efficacité est dépendante d'un équilibre entre les facteurs d'agression et les nombreux mécanismes de défense dont cet épithélium dispose.

Parmi ces mécanismes, l'épithélium de surface joue un rôle clef dans des mécanismes de défense tels que la réaction inflammatoire, la réponse immunitaire, le transport transépithélial d'électrolytes et la sécrétion de molécules à activité anti-infectieuse. La plupart de ces fonctions exigent le maintien d'une interaction et d'une polarité cellulaire. Toute altération de l'épithélium peut entraîner des dysfonctionnements de ces mécanismes de défense.

Ainsi, au cours des phénomènes inflammatoires bronchiques, aigus ou chroniques, une altération de l'efficacité du transport mucociliaire, en relation avec l'inhalation de nombreux agents infectieux (bactéries, virus,...) ou non infectieux (particules, pollution atmosphérique ou professionnelle), peut induire une altération de l'intégrité de la barrière épithéliale et entraîner des lésions plus ou moins importantes de l'épithélium respiratoire. Ces lésions peuvent varier depuis la perte de la jonctionnalité de la barrière épithéliale jusqu'à une desquamation épithéliale complète.

La réépithélialisation de la surface de l'épithélium respiratoire après une lésion est dans un premier temps dépendante de l'étalement et de la migration des cellules autour de la zone lésée (Zahm et al, Am J Respir Cell Mol Biol 1991, 5 :242-248 ; Hérard et al, Am J Respir Cell Mol Biol 1996, 15 : 624-632). Cette réépithélialisation doit s'accompagner d'une restauration de la fonction de défense de la barrière épithéliale.

Les complexes jonctionnels des cellules épithéliales jouent également un rôle prépondérant dans le maintien de l'intégrité épithéliale.

La fonction de barrière épithéliale est assurée par trois principaux types de complexes jonctionnels :
1) les jonctions serrées et les jonctions intermédiaires qui forment des ceintures d'ancrage intercellulaires,
2) les desmosomes qui sont situés à l'interface entre les cellules basales et les cellules cylindriques de surface et
3) les jonctions communicantes qui sont des voies de transduction de signaux intracellulaires.

Les jonctions serrées forment une barrière sélective régulant le passage des ions et molécules à travers l'espace paracellulaire. En microscopie électronique, on constate que les jonctions serrées forment une série de points de fusion entre les feuillets externes des membranes plasmiques de deux cellules adjacentes. Ces zones de contact membranaire apparaissent, par cryofracture, sous forme d'un réseau continu qui entoure l'apex de chaque cellule: Ce réseau est constitué de polymères de protéines membranaires, les claudines et l'occludine qui sont elles-mêmes reliées à des protéines cytoplasmiques parmi lesquelles on trouve notamment les protéines zonula occludens (ZO-1 à 3). L'occludine, les claudines ainsi que les ZO-1 sont souvent la cible des toxines bactériennes, conduisant à une altération de l'organisation et de la fonction des jonctions serrées (Coraux et al, Am J Respir Cell Mol Biol 2004, 5 :605-612).

Parmi les complexes jonctionnels, les jonctions communicantes sont impliquées dans la transmission de molécules d'une cellule à l'autre. Elles sont constituées d'un assemblage hiérarchisé de connexines qui sont regroupées en une unité hexamérique, le connexon. Chaque connexon est associé au connexon d'une cellule voisine pour former un tunnel de 2 à 4 nm permettant le passage de molécules de petite taille.

En plus de ces complexes jonctionnels assurant la protection de l'épithélium, et afin de prévenir la colonisation et l'infection par des micro-organismes inhalés, l'épithélium respiratoire maintient, grâce à la production de peptides et de molécules à activité bactéricide, un environnement stérile. Parmi ces agents anti-bactériens sécrétés par les cellules épithéliales respiratoires, il a été montré que l'inhibiteur de leucoprotéinase (SLPI) était capable d'induire la mort de bactéries gram-négatives et gram-positives.

Bien que l'altération des fonctions de défense des jonctions serrées soit initialement la conséquence du processus infectieux, il est également admis que l'inflammation importante qui accompagne l'infection peut être à l'origine des modifications de perméabilité de la barrière épithéliale.

Les cytokines représentent une famille de molécules polypeptidiques solubles de faible masse moléculaire libérées par de très nombreuses cellules activées au cours des processus immunitaires et inflammatoires. Parmi ces cytokines, l'interleukine 8 (IL-8) est augmentée dans les maladies inflammatoires et infectieuses. Le pouvoir chemo-attractant de l'IL-8 vis à vis des neutrophiles constitue un mécanisme de défense adapté à la plupart des situations pathologiques, mais une réponse exagérée des neutrophiles peut exacerber l'altération des fonctions de défense de l'épithelium respiratoire (Tirouvanziam et al, Am J Respir Cell Mol Biol, 2000 ,2 :121-127).

La restauration de ces fonctions de défense après une agression de l'épithélium est donc une étape importante dans le maintien des fonctions de barrière de l'épithélium et la mise en évidence de molécules susceptibles de favoriser cette restauration représente donc un enjeu important.

Actuellement le traitement des agressions de l'épithélium consiste surtout en un traitement symptomatique : bronchodilatateurs, anti-inflammatoires, antibiotiques, muco-régulateurs.

Le brevet français FR2 847 818 décrit un acide hyaluronique, son mode de préparation et son application dans une composition présentant une activité thérapeutique vis à vis notamment des affections respiratoires des voies aériennes supérieures.

L'acide hyaluronique décrit dans ce brevet présente un poids moléculaire d'au plus 10⁵ Da. L'acide hyaluronique est notamment utilisé dans le cadre du traitement des affections respiratoires des voies aériennes supérieures (réparation de l'épithélium respiratoire).

Le brevet US 6,806,259 décrit une préparation d'acide hyaluronique de poids moléculaire de 50 000 à 200 000 Daltons. Cette préparation est administrée par voie orale en tant que complément nutritionnel pour adoucir la peau humaine.

La demande US 2006/166930 concerne une composition pharmaceutique contenant de l'acide hyaluronique acétylé pour le traitement de la sécheresse de l'oeil. Le poids moléculaire de l'acide hyaluronique acétylé est préférentiellement de 10 000 à 1 000 000 Daltons.

Le brevet US 5,166,331 décrit deux fractions d'acide hyaluronique, l'une de poids moléculaire de 50 000 à 100 000 (hyalastine) utilisée pour la cicatrisation et l'autre de poids moléculaire de 500 000 à 730 000 (hyalectine) utilisée pour des injections intra oculaires et intra articulaires.

Les demandes DE 10 360 425, WO 03/053452 et WO 03/049747 concernent l'utilisation d'acide hyaluronique de poids moléculaire de 50 000 à 10 000 000 Daltons, de sel d'acide hyaluronique et/ou leurs dérivés pour la production d'une composition pharmaceutique pour le traitement des complications ophtalmiques et/ou rhiniques.

Aucun de ces documents, utilisant l'acide hyaluronique ne décrit la restauration des fonctions de défense des complexes jonctionnels après une agression de l'épithélium et jusqu'à aujourd'hui, seuls des cytokines comme par exemple le TGF-béta ou des glucocortocoïdes ont été décrits comme étant capable d'augmenter l'expression des molécules d'adhérence intercellulaires.

Un des buts de l'invention est l'utilisation d'acide hyaluronique de bas poids moléculaire, de 30 000 à 45 000 Daltons pour stimuler les mécanismes impliqués dans la restauration des fonctions de défense et présentant une activité de protection et/ou d'amélioration des fonctions de protection de la muqueuse respiratoire, de la muqueuse intestinale, ou de l'oeil, dans le cas d'agressions provenant d'agents physiques, chimiques ou microbiologiques.

Un autre but de l'invention est de fournir des compositions pharmaceutiques contenant de l'acide hyaluronique pour la préparation d'un médicament destiné à la prévention de pathologies choisies parmi l'asthme, les allergies respiratoires, le syndrome de détresse respiratoire.

Un autre but de l'invention est de fournir des compositions contenant de l'acide hyaluronique destinées à l'amélioration de la fonction de protection de l'oeil dans le cadre de troubles oculaires.

Un autre but de l'invention est de fournir des compositions alimentaires contenant de l'acide hyaluronique destinée à l'amélioration de la fonction de protection de la muqueuse intestinale dans le cadre des troubles digestifs.

Par conséquent, la présente invention concerne l'utilisation d'au moins un acide hyaluronique de 30 000 à 45 000 Daltons de poids moléculaire, préférentiellement 40 000 Daltons, ou de ses sels correspondants, pour la préparation de compositions destinées à la protection et/ou la restauration de l'intégrité de la muqueuse des voies aériennes supérieures et inférieures, de la muqueuse intestinale, ou de l'oeil, dans le cas d'agressions provenant d'agents physiques, chimiques ou microbiologiques, et dans lesquelles les cellules épithéliales sont impliquées, en particulier les complexes jonctionnels de celles-ci.

En deçà de 30 000 Daltons, l'acide hyaluronique devient inflammatoire (US5,166,331) et au-delà de 45000, l'acide hyaluronique perd son activité.

L'expression « sels correspondants » désigne les sels de sodium, potassium, lithium, calcium, barium, stontium, magnésium, aluminium, ammonium ou ammonium substitué.

L'expression « muqueuse respiratoire » désigne l'épithélium des voies aériennes supérieures et inférieures, qui est pseudo stratifié, et constitué des cellules ciliées, de cellules caliciformes, de cellules basales, de cellules en brosse et de cellules neuro-endocrines, ainsi que le chorion qui est très vascularisé et comprend de nombreuses fibres élastiques.

L'expression « voies aériennes supérieures » comprend les fosses nasales, le pharynx et le larynx et l'expression « voies aériennes inférieures » comprend la trachée, les bronches et les bronchioles.

L'expression « muqueuse intestinale » désigne la partie intérieure du tube digestif et comprend :
- l'épithélium qui est formé, à partir de l'estomac, d'une seule couche de cellules reliées les unes aux autres par des jonctions occlusives au niveau du bord de leur surface apicale; des cellules exocrines qui sécrètent du mucus dans la lumière ainsi que des cellules endocrines qui libèrent des hormones dans le sang, sont incluses dans la couche épithéliale,
- la lamina propria, couche de tissu conjonctif lâche très riche en capillaires sanguins et lymphatiques, en leucocytes et autres cellules du système de défense contre les micro-organismes qui sont susceptibles de traverser l'épithélium,
- la muscularis mucosae fine couche de cellules musculaires lisses qui en se contractant peuvent modifier les plis de l'épithélium.

L'expression « agents physiques » désigne de façon générale des sources d'énergie capables de causer des lésions ou des maladies. Ce sont notamment le bruit, les vibrations, les rayonnements (ionisants, tels que les rayons X et gamma ou optiques tels que rayonnements ultraviolets, rayonnements lasers et lampes infrarouges), les ondes (par exemple, micro-ondes ou radiofréquence) et les températures et pressions extrêmes.

L'expression « agents chimiques» désigne les produits chimiques (non dangereux, dangereux, dangereux non CMR (cancérogène mutagènes reprotoxiques), dangereux CMR) ainsi que les composés contenu dans la pollution atmosphérique (les poussières, le SO₂, les NOx, le CO, les métaux lourds, les composés organiques volatils, le fluor, l'acide chlorhydrique, etc..., les gaz à effet de serre tels que le CO₂, le CH₄, le N₂O, les CFC, HFC, PFC et SF₆ et d'autres substances telles que l'ozone, les organochlorés (dioxines et furannes), les HAP (Hydrocarbures Aromatiques Polycycliques), etc.

L'expression « agents microbiologiques» désigne des agents présents dans l'air, la nourriture et l'eau potable, notamment les bactéries, les protozoaires, les virus et les champignons, et peuvent être à l'origine de diverses maladies.

Selon un mode de réalisation préféré, la concentration de l'acide hyaluronique utilisé ci-dessus, est de 0,1 g/l à 4g/l, et préférentiellement de 0,2 à 1g/l.
En deçà et au delà de cette concentration, l'acide hyaluronique devient inactif. Selon un autre mode de réalisation préféré, la présente invention concerne l'utilisation d'au moins un acide hyaluronique défini ci-dessus pour la préparation d'un médicament destiné au traitement et/ou la prévention de pathologies provoquées par les agressions provenant d'agents physiques, chimiques ou microbiologiques.

Selon un mode de réalisation particulièrement préféré, la présente invention concerne l'utilisation d'un acide hyaluronique défini ci-dessus, pour la préparation de compositions cosmétiques et alimentaires.

Selon un autre mode de réalisation, l'acide hyaluronique défini ci-dessus est utilisé pour la préparation d'un médicament destiné à la prévention de pathologies telles que l'asthme, les allergies respiratoires, le syndrome de détresse respiratoire, dans lesquelles les cellules épithéliales impliquées sont des cellules de la muqueuse des voies aériennes supérieures et inférieures.

L'asthme est une maladie des bronches qui, lors de crises, entraîne des difficultés à inspirer et surtout à expirer l'air des poumons. Les crises peuvent être déclenchées par différents facteurs comme l'effort, l'humidité ou la poussière ou d'autres allergènes tels que les polluants atmosphériques.

Les allergies respiratoires sont des maladies déclenchées par des allergènes tels que les acariens qui sont responsables de 70 à 80 % des asthmes allergiques de l'enfant.

Parmi les autres pneumallergènes (allergènes qui pénètrent dans l'organisme par voie respiratoire) de l'environnement intérieur, les animaux domestiques (chat, chien, rongeurs), les blattes, les moisissures, sont une source fréquente d'allergie respiratoire.

Dans l'environnement extérieur, les polluants atmosphériques, les moisissures atmosphériques peuvent également être en cause.

Des allergènes professionnels peuvent également être responsables de sensibilisations et d'allergies respiratoires (sources les plus fréquentes : farine, animaux de laboratoire, latex...).

Le syndrome de détresse respiratoire est une détresse respiratoire mettant en jeu le pronostic vital, due à une atteinte pulmonaire aiguë. Il est provoqué par différents facteurs tels que l'aspiration d'hydrocarbures, l'inhalation de composés irritants (chlore, NO₂, fumées, ozone,oxygène à haute concentration, fumées métalliques, gaz moutarde), des herbicides tels que le paraquat ou des opiacés (tels que l'héroine, la morphine, le dextropropoxyphène, ou la méthadone).

L'exemple 1 ci-après montre que l'acide hyaluronique de l'invention provoque une augmentation de la fonctionnalité des jonctions communicantes et permet donc ainsi de prévenir les maladies notamment provoquées par les polluants atmosphériques.

Selon un autre mode de réalisation, l'acide hyaluronique défini ci-dessus est utilisé pour la préparation d'une composition destinée à l'amélioration de la fonction de protection de la muqueuse intestinale dans le cadre des troubles digestifs, en particulier les gastro-entérites, les nécroses ischémiques et les ulcérations de la muqueuse intestinale, dans lesquelles les cellules épithéliales impliquées sont des cellules de la muqueuse intestinale

Les troubles digestifs regroupent de multiples symptômes qui peuvent toucher tous les organes du tube digestif. Les désordres digestifs observés ont pour origine un dysfonctionnement de ces organes.

Les gastro-entérites sont des infections inflammatoires caractérisées par l'émission brutale et fréquente de selles liquides et abondantes.

La nécrose ischémique est une nécrose de coagulation par défaut de vascularisation entraînant la momification des éléments cellulaires présents.

Les ulcérations de la muqueuse intestinale entraînent la destruction de la muqueuse intestinale par invasion de l'épithélium intestinal.

Selon un autre mode de réalisation, l'acide hyaluronique défini ci-dessus est utilisé pour la préparation d'une composition destinée à l'amélioration de la fonction de protection de l'oeil dans le cadre de troubles oculaires, en particulier le stress hydrique, la sécheresse de l'oeil, les lésions de la cornée et les kératopathies, dans lesquelles les cellules épithéliales impliquées sont des cellules de l'oeil, et notamment de la cornée.

L'expression « stress hydrique » désigne un déficit en eau du globe oculaire.

Si les larmes sont déficientes en certains des composants importants tels que le sébum ou que leur production est insuffisante, le film lacrymal peut se dégrader. Il se forme alors des points secs sur la cornée causant les symptômes de la sécheresse oculaire : démangeaisons, sensations de brûlure, de corps étrangers et inconfort général.

L'expression « lésions de la cornée » désigne un défaut superficiel de l'épithélium de la cornée provoqué par abrasion ou frottement, habituellement dues à un traumatisme ou corps étranger dans l'oeil.

Le terme « kératopathies » désigne toutes les maladies intéressant la cornée, d'origine traumatique, chimique, infectieuse ou génétique. Les maladies de cornée sont très nombreuses et se traduisent par une perte de transparence pouvant entraîner une perte plus ou moins complète et durable de la vision selon la cause et le siège des lésions.

Dans encore un autre aspect, la présente invention concerne une composition pharmaceutique comprenant au moins un acide hyaluronique de 30 000 à 45 000 de poids moléculaire, préférentiellement 40 000 Daltons, ou ses sels correspondants à une concentration de 0,1 g/l à 1g/l en association avec un véhicule pharmaceutiquement acceptable.

Dans un mode de réalisation préféré, la composition pharmaceutique ci-dessus définie est formulée pour être administrée par voie topique à une dose comprise de 50 mg/j à 300 mg/j, préférentiellement de 100 mg/j à 200 mg/j ou à une dose par voie orale comprise de 0.66 mg/kg/j à 4 mg/kg/j, préférentiellement de 1.33 mg/kg/j à 2.66 mg/kg/j.

Selon un autre mode de réalisation, la composition pharmaceutique ci-dessus définie se présente sous forme gastrorésistante.

L'expression « forme gastrorésistante » se réfère à une composition qui est sous forme de véhicule gastrorésistant, c'est-à-dire que l'acide hyaluronique est protégé de l'acidité de l'estomac.

Le véhicule gastrorésistant physiologiquement stable est choisi parmi: les microgranules gastrorésistantes, les microgranules pelliculées gastrorésistantes, les nanoparticules, ou nanosphères, gastrorésistantes, les microsphères gastrorésistantes, les microcapsules gastrorésistantes, les granulés gastrorésistants, les granulés pelliculés gastrorésistants, les liposomes gastrorésistants, les liposomes pelliculés gastrorésistants, les lyocs gastrorésistants, les lyocs pelliculés gastrorésistants, les pompes osmotiques dans un enrobage gastrorésistant, les gommes, les sphéroïdes gastrorésistants, les sphérules gastrorésistantes, les sphéroïdes pelliculés gastrorésistants, les sphérules pelliculées gastrorésistantes, les comprimés pelliculés gastrorésistants, les gélules pelliculées gastrorésistantes.

Dans un autre aspect, la présente invention concerne une composition alimentaire comprenant un acide hyaluronique de 30 000 à 45 000 de poids moléculaire, préférentiellement 40 000 Daltons, ou ses sels correspondants à une concentration de 0,1 g/l à 1g/l.

Dans un mode de réalisation préféré, la composition alimentaire ci-dessus définie est formulée pour être administrable par voie orale à une dose comprise de 50 mg/j à 300 mg/j, préférentiellement de 100 mg/j à 200 mg/j.

Un exemple de composition alimentaire est présenté dans l'exemple 4.

Dans un mode de réalisation préféré, la composition alimentaire ci-dessus définie se présente sous forme gastrorésistante.

Une composition alimentaire gastrorésistante peut se présenter sous forme de comprimés, de gélules, de sachets, ou de granulés gastrorésistants.

### DESCRIPTION DES FIGURES :

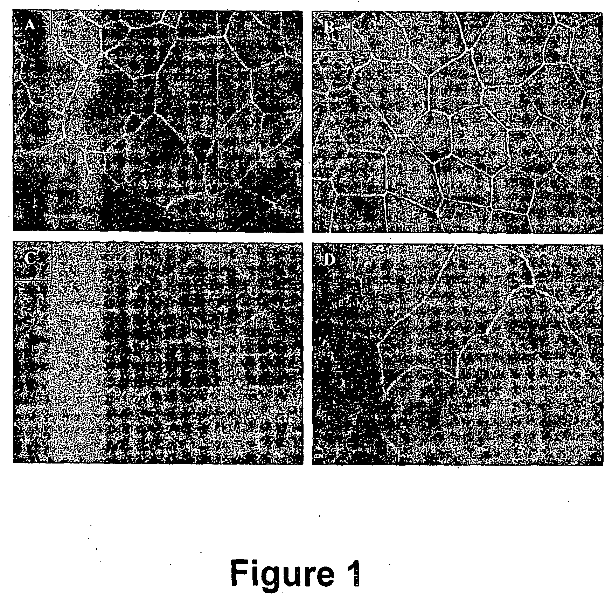

La figure 1 représente l'immunolocalisation de la protéine jonctionnelle ZO-1. Dans les zones de cellules confluentes, la localisation de ZO-1 n'est pas différente selon que les cellules ont été incubées ou non avec de l'acide hyaluronique, mais le réseau formé par ZO-1 est plus dense lorsque les cellules ont été incubées avec l'acide hyaluronique (A,B). Par contre au niveau des cellules situées en périphérie de culture et qui sont dédifférenciées, on observe que l'expression de ZO-1 est plus importante et plus continue lorsque les cellules sont incubées avec de l'acide hyaluronique (D), comparativement aux cellules non-incubées avec l'acide hyaluronique (C). Le même profil d'expression est observé pour la protéine jonctionnelle occludine.

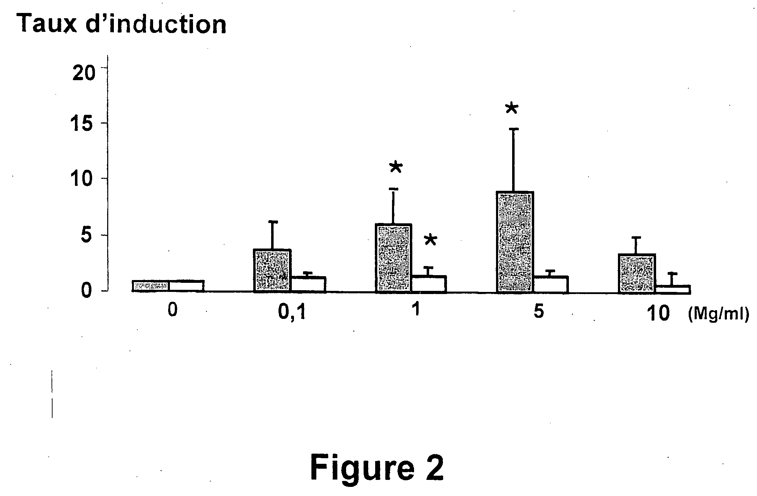

La figure 2 représente l'effet de la concentration en acide hyaluronique (axe des abscisses) sur l'expression membranaire des protéines ZO-1 et occludine (axe des ordonnées). Les colonnes en blanc correspondent à l'occludine et les colonnes en gris à ZO-1. Les étoiles indiquent une différence statistiquement significative (p<0,05) par rapport au témoin sans acide hyaluronique.

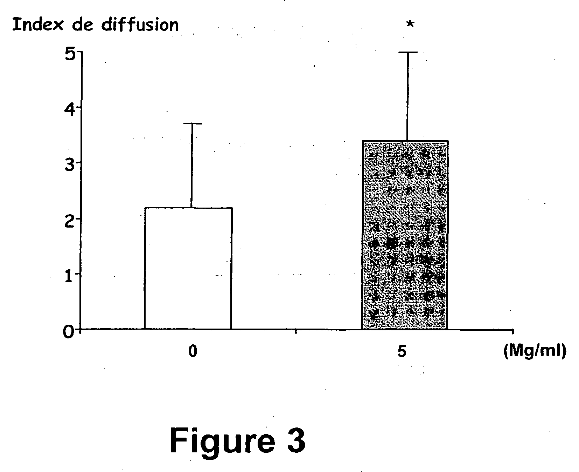

La figure 3 représente l'effet de l'acide hyaluronique (axe des abscisses) sur la fonctionnalité des jonctions communicantes (axe des ordonnées). La colonne en blanc correspond au témoin sans acide hyaluronique et la colonne en gris correspond à l'acide hyaluronique à 5 mg/ml. L'étoile indique un résultat statistiquement significatif (p<0,05) par rapport au témoin.

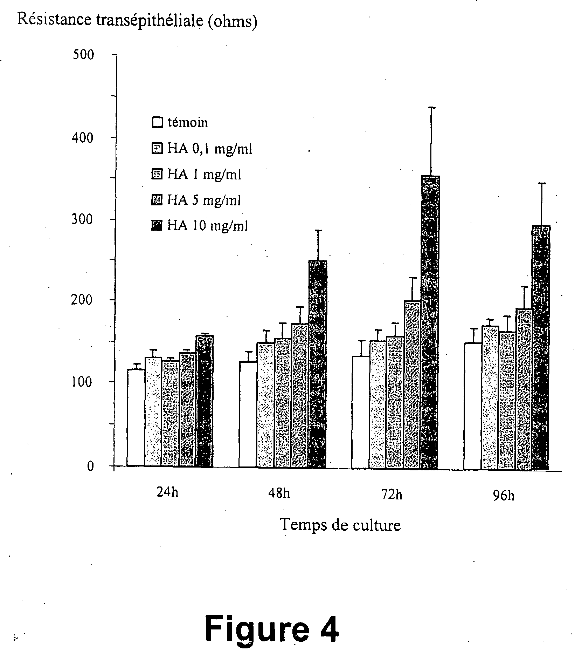

La figure 4 représente l'effet de la concentration en acide hyaluronique et du temps de culture des cellules épithéliales respiratoires (axe des abscisses) sur la résistance transépithéliale (axe des ordonnées). Une augmentation significative (p<0,02) de la résistance transépithéliale est observée en fonction du temps et de la concentration en acide hyaluronique.

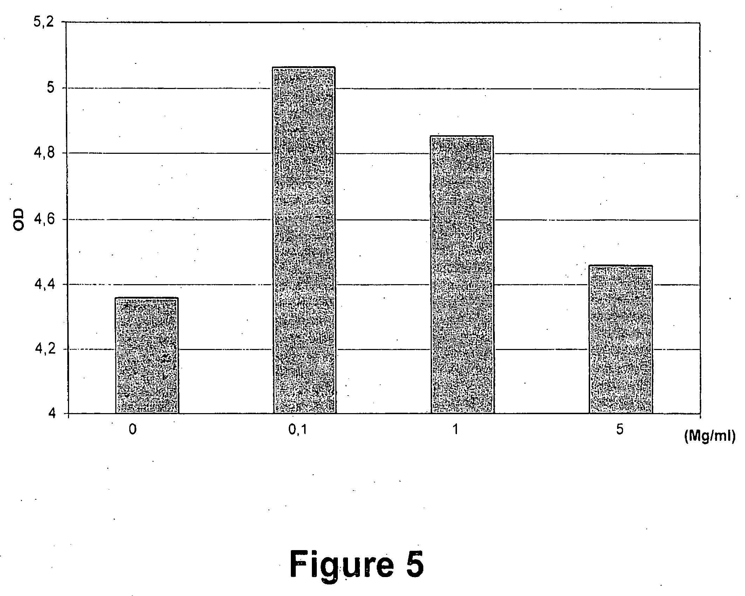

La figure 5 représente l'effet de la concentration en acide hyaluronique (axe des abscisses) sur l'expression des protéines occludines de cellules de peau (axe des ordonnées). Les diagrammes correspondent aux mesures d'intensité de l'expression de l'activité de la peroxydase. Les valeurs sont les moyennes de trois points.

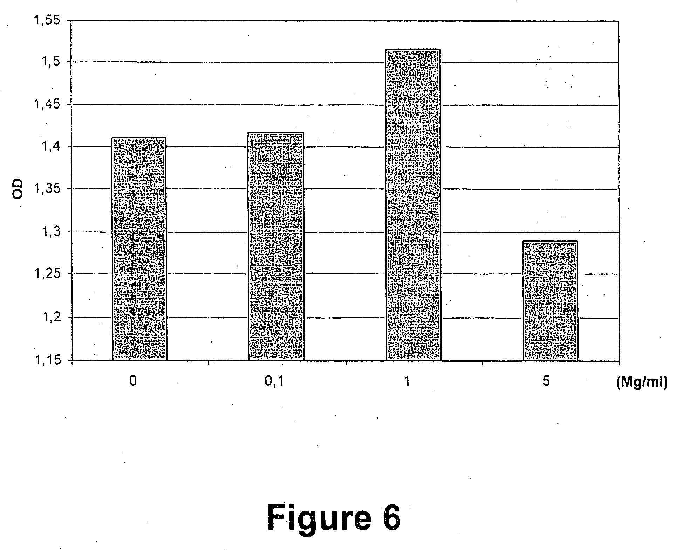

La figure 6 représente l'effet de la concentration en acide hyaluronique (axe des abscisses) sur l'expression des protéines ZO-1 de cellules de peau (axe des ordonnées). Les diagrammes correspondent aux mesures d'intensité de l'expression de l'activité de la peroxydase. Les valeurs sont les moyennes de trois points.

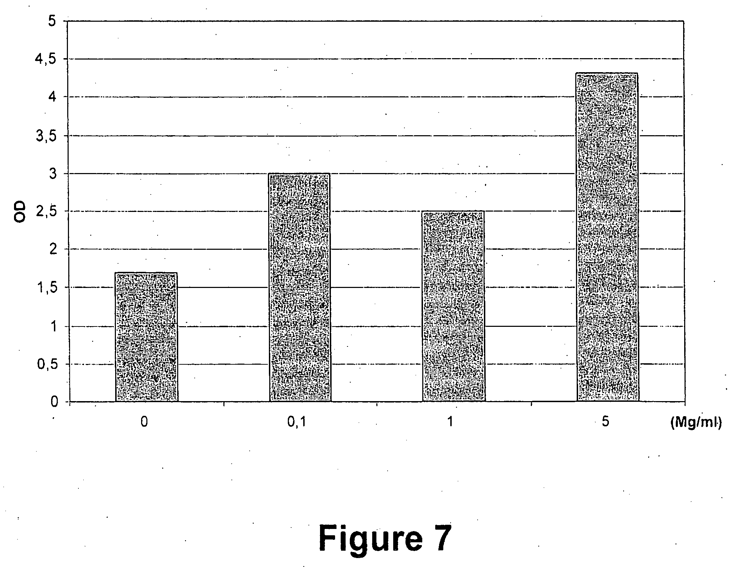

La figure 7 représente l'effet de la concentration en acide hyaluronique (axe des abscisses) sur l'expression des protéines occludines de cellules intestinales (axe des ordonnées). Les diagrammes ci-après correspondent aux mesures d'intensité de l'expression de l'activité de la peroxydase. Les valeurs sont les moyennes de trois points.

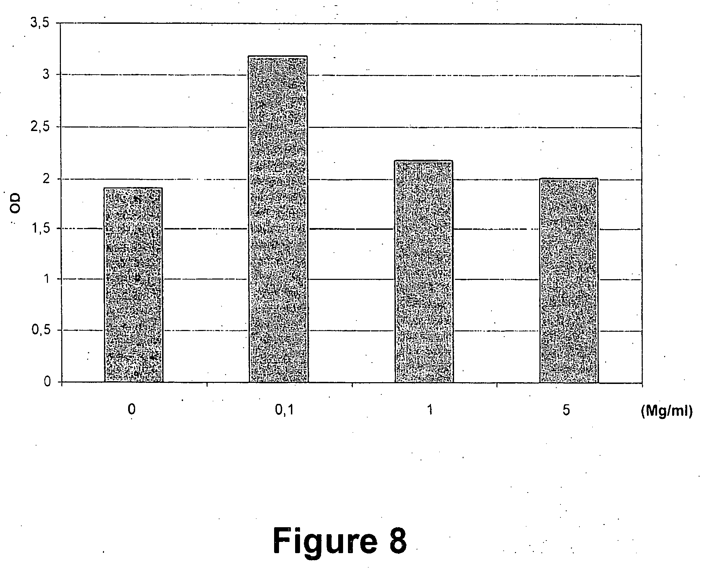

La figure 8 représente l'effet de la concentration en acide hyaluronique (axe des abscisses) sur l'expression des protéines ZO-1 de cellules intestinales. Les diagrammes ci-après correspondent aux mesures d'intensité de l'expression de l'activité de la peroxydase. Les valeurs sont les moyennes de trois points.

### PARTIE EXPERIMENTALE

### Exemple 1 : Exemple d'augmentation sur des cellules épithéliales respiratoires de l'expression des protéines impliquée dans le maintien de l'intégrité épithéliale par l'utilisation de l'acide hyaluronique décrit dans la présente invention.

Des cellules épithéliales respiratoires sont cultivées dans des anneaux de restriction contenant du milieu DMEM/F12 supplémenté avec des antibiotiques, des facteurs de croissance et avec différentes concentrations d'acide hyaluronique (0,1, 1, 5 ou 10 mg/ml) ou en absence d'acide hyaluronique. A confluence, les anneaux de restriction sont retirés pour permettre la migration des cellules en périphérie de la zone de culture. Les cellules sont ensuite fixées puis incubées successivement avec les anti-corps anti-ZO-1 ou anti-Occludine, puis avec un anti-corps biotinylé et enfin avec de la steptavidine couplée à de l'Alexa Fluor 488 (Invitrogen). Les préparations sont montées sur une lame de verre dans une solution permettant de prévenir le photo-blanchiment et observées à l'aide d'un microscope à fluorescence à un grossissement de x40 afin de visualiser la localisation cellulaire des protéines ZO-1 et occludine.

Dans une autre série d'expérimentations, deux extractions différentes ont été réalisées sur des cultures de cellules: une extraction de protéines totales et une extraction de protéines membranaire afin d'évaluer l'expression de ZO-1 et d'occludine par technique de Western Blot.

La fonctionnalité des jonctions serrées a été évaluée par la mesure de la résistance transépithéliale : les cellules épithéliales respiratoires sont cultivées dans une boite de culture à double compartiment permettant la mesure de la résistance épithéliale. Cette mesure est réalisée toutes les 24h à l'aide d'une double électrode qui permet d'établir une tension constante entre le milieu apical et le milieu basal de la chambre de culture. La mesure du courant induit permet de calculer la résistance de la couche de cellules. L'augmentation de la résistance trans-épithéliale traduit la présence de jonctions serrées fonctionnelles.

La fonctionnalité des jonctions communicantes a été évaluée en utilisant des techniques de vidéo-microscopie et une technique de FRAP (fluorescence recovery after photobleaching) : mesure de la diffusion d'une sonde fluorescente via les jonctions communicantes. (Abaci M et al, Biotechnol J, 2007,2 : 50-61 ; Tedelind S, Eur J Endocrinol 2003, 149 :215-221)

### Résultats :

### Effet de l'acide hyaluronique sur l'expression des protéines de défense

### Expression de ZO-1 et Occludine evaluée par immunocytochimie

Dans les zones de cellules confluentes, la localisation de ZO-1 -n'est pas différente selon que les cellules ont été incubées ou non avec de l'acide hyaluronique. Par contre au niveau des cellules situées en périphérie de culture et qui sont dédifférenciées, on observe que l'expression de ZO-1 est plus importante et plus continue lorsque les cellules sont incubées avec de l'acide hyaluronique, comparativement aux cellules non incubées avec l'acide hyaluronique (figure 1).

Le même profil d'expression est observé pour la protéine jonctionnelle occludine.

### Expression de ZO-1 et Occludine évaluée par Western Blot

Les taux d'expression au niveau membranaire des protéines jonctionnelles ZO1 et occludine en présence de concentrations croissantes d'acide hyaluronique sont représentés dans la figure 2. La quantité de protéine mesurée en présence d'acide hyaluronique est exprimée en fonction de la quantité de protéine mesurée en absence d'acide hyaluronique. Les valeurs représentées correspondent à la moyenne ± erreur standard de 4 expérimentations.

On observe une augmentation significative (p< 0,05) de l'expression de la protéine ZO1 lorsque les cellules sont incubées en présence d'acide hyaluronique à la concentration de 1 et 5 mg/ml. Une augmentation significative (p< 0,05) de l'expression d'occludine est également notée lorsque les cellules sont incubées en présence d'acide hyaluronique à la concentration de 1 mg/ml.

### Analyste de la fonctionnalité des jonctions communicantes

La fonctionnalité des jonctions communicantes a été évaluée grâce à la mesure de la diffusion intercellulaire d'une molécule fluorescente. La figure 3 montre que l'incubation des cellules épithéliales respiratoires en présence d'acide hyaluronique (5 mg/ml) induit une augmentation significative (p < 0,05) de l'index de diffusion, ce qui traduit une augmentation de la fonctionnalité des jonctions communicantes.

### Exemple 2 (exemple de référence): Exemple d'augmentation sur des cellules de peau de l'expression des protéines impliquée dans le maintien de la contiguïté cellulaire par l'utilisation de l'acide hyaluronique décrit dans la présente invention.

Des kératinocytes normaux humains issus d'explant de chirurgie plastique (Normal Humain Epidermal Keratinocytes ; NHEK) sont incubés dans un milieu SFM medium (invitrogen 17005075) complémenté des facteurs de croissance tels que EGF (Epidermal Growth Factor) 0,25 ng/ml, extrait d'hypophyse 25µg/ml (Invitrogen 37000015), gentamicine 25µg/ml (Sigma G1397), 24 heures à 37°C et sous 5% de CO₂ puis le milieu est éliminé et les cellules sont mises en présence ou non (contrôle) de la molécule d'acide hyaluronique de l'invention. Les concentrations en acide hyaluronique testées sont 0,1 mg/ml, 1mg/ml et 5 mg/ml.

Après 72 heures d'incubation à 37°C et 5% de CO₂, le milieu de culture est éliminé puis les cellules sont rincées avec un tampon phosphate puis immédiatement congelées à -80°C.

Les protéines totales exprimées par les cellules sont extraites puis mises en contact avec un anticorps anti ZO1 (Cliniscience 33-9100) et anti-occludine Cliniscience 33-1500) couplé à un système de détection faisant intervenir un deuxième anticorps conjugué avec une peroxydase. L'expression de la coloration en présence du substrat de la peroxydase renseigne sur l'expression des marqueurs ZO1 et occuldine en fonction de la quantité de HA présent dans le milieu.

Sur les figures 4 et 5 on observe une augmentation de l'expression des protéines jonctionnelles occludine et ZO-1 lorsque les cellules sont incubée avec de l'acide hyaluronique de l'invention.
A noter que le maximum d'expression est obtenu à une concentration de 0,1mg/ml pour l'occludine et 1 mg/ml pour la protéine ZO-1.

La peau, qui est un des organes le plus exposé aux agressions multiples de l'environnement, peut être à même de mieux résister aux agressions diverses telles que la pollution atmosphérique lorsque cette dernière est en contact avec un HA de faible poids moléculaire permettant d'augmenter la cohésion entre les cellules.

### Exemple 3 : Exemple d'augmentation sur des cellules intestinales de l'expression des protéines impliquée dans le maintien de la contiguïté cellulaire par l'utilisation de l'acide hyaluronique décrit dans la présente invention.

Des cellules de colon humain (Epithelial Human Caucasian Colon adenocarcinoma R51, Caco-2) sont incubées dans un milieu MEM (invitrogen 21090-022) complémenté d'acides aminés non essentiels (Invitrogen 11140-035), Glutamine 2mM (Invitrogen 25030024), pénicilline 50UI/ml, streptomycine 50 µg/ml (invitrogen 15070063) sérum de veau foetal 10% (FCS, invitrogen 10270098) 24 heures à 37°C et sous 5% de CO₂ puis le milieu est éliminé et les cellules sont mises en présence ou non (contrôle) de la molécule d'acide hyaluronique de l'invention. Les concentrations en acide hyaluronique testées sont 0,1 mg/ml, 1mg/ml et 5 mg/ml.

Apres 72 heures d'incubation à 37°C et 5% de CO₂, le milieu de culture est éliminé puis les cellules sont rincées avec un tampon phosphate puis immédiatement congelées à -80°C.

Les protéines totales exprimées par les cellules sont extraites puis mises en contact avec un anticorps anti ZO1 (Cliniscience 33-9100) et anti-occludine Cliniscience 33-1500) couplé à un système de détection faisant intervenir un deuxième anticorps conjugué avec une peroxydase. L'expression de la coloration en présence du substrat de la peroxydase renseigne sur l'expression des marqueurs ZO1 et occuldine en fonction de la quantité de HA présent dans le milieu.

Sur les figures 6 et 7 il apparaît que l'expression de protéines jonctionnelles occludine et ZO1 est modulée par la présence de l'acide hyaluronique de l'invention.

L'occludine est exprimée en plus grande quantité à haute concentration d'acide hyaluronique alors qu'en ce qui concerne la protéine ZO1, l'expression maximale est observée à une concentration de 0,1 mg/ml.

Le contact entre les cellules du tractus digestif par ingestion d'acide hyaluronique de bas poids moléculaire par exemple, permet l'augmentation de l'expression des protéines jonctionnelles ce qui traduit un renforcement des fonctions des de défense joué par la barrière épithéliale.

### Exemple 4 : Une composition alimentaire destinée à la muqueuse intestinale contenant la molécule de l'invention est représentée par la formule suivante :

| | |
|---|---|
| Polymère de l'invention | 0,01% à 0,1% |
| Stéarate de magnésium | 0,1% |
| Excipient : amidon de riz | QSP1 gramme |
| Capsule | Gélatine |

## Revendications

1. Utilisation d'au moins un acide hyaluronique de 30 000 à 45 000 Daltons de poids moléculaire, préférentiellement 40 000 Daltons, ou de ses sels correspondants, pour la préparation de compositions destinées à l'amélioration de la fonction de protection de la muqueuse intestinale dans le cadre de pathologies de la muqueuse intestinale choisies parmi les gastro-entérites, les nécroses ischémiques et les ulcérations de la muqueuse intestinale, l'amélioration de la fonction de protection de l'oeil dans le cadre des troubles oculaires choisis parmi le stress hydrique, la sécheresse de l'oeil, les lésions de la cornée et les kératopathies et dans lesquelles les cellules épithéliales sont impliquées.

2. Utilisation d'au moins un acide hyaluronique de 30 000 à 45 000 Daltons de poids moléculaire, préférentiellement 40 000 Daltons, ou de ses sels correspondants, pour la préparation d'un médicament destiné à la prévention des pathologies des voies aériennes supérieures et inférieures choisies parmi l'asthme, les allergies respiratoires et le syndrome de détresse respiratoire.

3. Utilisation d'un acide hyaluronique selon la revendication 1, à une concentration de 0,1 g/l à 4g/l, préférentiellement de 0,2 à 1g/l.

4. Utilisation d'un acide hyaluronique selon l'une des revendications 1 à 3, **caractérisée en ce que** la composition est une composition alimentaire.

5. Composition pharmaceutique, comprenant au moins un acide hyaluronique de 30 000 à 45 000 de poids moléculaire, préférentiellement 40 000 Daltons, ou ses sels correspondants, formulée pour être administrée par voie topique à une dose comprise de 50 mg/j à 300 mg/j, préférentiellement de 100 mg/j à 200 mg/j ou à une dose par voie orale comprise de 0,66 mg/kg/j à 4 mg/kg/j, préférentiellement de 1,33 mg/kg/j à 2,66 mg/kg/j pour son utilisation dans l'amélioration de la fonction de protection de la muqueuse intestinale dans le cadre de pathologies de la muqueuse intestinale choisies parmi les gastro-entérites, les nécroses ischémiques et les ulcérations de la muqueuse intestinale, l'amélioration de la fonction de protection de l'oeil dans le cadre des troubles oculaires choisis parmi le stress hydrique, la sécheresse de l'oeil, les lésions de la cornée et les kératopathies et dans lesquelles les cellules épithéliales sont impliquées.

6. Composition pharmaceutique pour son utilisation selon la revendication 5, sous forme gastrorésistante.

7. Composition alimentaire comprenant au moins un acide hyaluronique de 30 000 à 45 000 de poids moléculaire, préférentiellement 40 000 Daltons, ou ses sels correspondants, formulée pour être administrable par voie orale à une dose comprise de 50 mg/j à 300 mg/j, préférentiellement de 100 mg/j à 200 mg/j, pour son utilisation dans l'amélioration de la fonction de protection de la muqueuse intestinale dans le cadre de pathologies de la muqueuse intestinale choisies parmi les gastro-entérites, les nécroses ischémiques et les ulcérations de la muqueuse intestinale, l'amélioration de la fonction de protection de l'oeil dans le cadre des troubles oculaires choisis parmi le stress hydrique, la sécheresse de l'oeil, les lésions de la cornée et les kératopathies et dans lesquelles les cellules épithéliales sont impliquées.

8. Composition alimentaire pour son utilisation selon la revendication 7, sous forme gastrorésistante.

## Patentansprüche

1. Verwendung mindestens einer Hyaluronsäure von 30000 bis 45000 Dalton, vorzugsweise 40000 Dalton, Molekulargewicht oder ihrer entsprechenden Salze für die Herstellung von Zusammensetzungen, die für die Verbesserung der Schutzfunktion der Darmschleimhaut im Rahmen von Pathologien der Darmschleimhaut, ausgewählt unter den Gastroenteritiden, den ischämischen Nekrosen und den Ulzerationen der Darmschleimhaut, die Verbesserung der Schutzfunktion des Auges im Rahmen von Augenstörungen, ausgewählt unter Wasserstress, Trockenheit des Auges, Verletzungen der Hornhaut und den Keratopathien, und bei denen die Epithelialzellen betroffen sind, bestimmt sind.

2. Verwendung mindestens einer Hyaluronsäure von 30000 bis 45000 Dalton, vorzugsweise 40000 Dalton, Molekulargewicht oder ihrer entsprechenden Salze für die Herstellung eines Medikaments, das für die Prävention der Pathologien der obere und unteren Luftwege, ausgewählt unter Asthma, respiratorischen Allergien oder Atemnot-Syndrom, bestimmt ist.

3. Verwendung einer Hyaluronsäure nach Anspruch 1 in einer Konzentration von 0,1 g/l bis 4 g/l, vorzugsweise von 0,2 bis 1 g/l.

4. Verwendung einer Hyaluronsäure nach einem der Ansprüche 1 bis 3, **dadurch gekennzeichnet, dass** die Zusammensetzung eine Lebensmittelzusammensetzung ist.

5. Pharmazeutische Zusammensetzung, umfassend mindestens eine Hyaluronsäure von 30000 bis 45000, vorzugsweise 40000 Dalton, Molekulargewicht oder ihrer entsprechenden Salze, die derart formuliert ist, dass sie auf topischem Weg in einer Dosis von 50 mg/d bis 300 mg/d, vorzugsweise von 100 mg/d bis 200 mg/d oder in einer Dosis auf oralem Weg zwischen 0,66 mg/kg/d bis 4 mg/kg/d, vorzugsweise von 1,33 mg/kg/d bis 2,66 mg/kg/d, für ihre Verwendung zur Verbesserung der Schutzfunktion der Darmschleimhaut im Rahmen von Pathologien der Darmschleimhaut, ausgewählt unter den Gastroenteritiden, den ischämischen Nekrosen und den Ulzerationen der Darmschleimhaut, die Verbesserung der Schutzfunktion des Auges im Rahmen von Augenstörungen, ausgewählt unter Wasserstress, Trockenheit des Auges, Verletzungen der Hornhaut und den Keratopathien, und bei denen die Epithelialzellen betroffen sind, verabreicht werden kann.

6. Pharmazeutische Zusammensetzung für ihre Verwendung nach Anspruch 5 in gastroresistenter Form.

7. Lebensmittelzusammensetzung, umfassend mindestens eine Hyaluronsäure von 30000 bis 45000, vorzugsweise 40000, Dalton Molekulargewicht, die derart formuliert ist, dass sie auf oralem Weg in einer Dosis von 50 mg/d bis 300 mg/d, vorzugsweise von 100 mg/d bis 200 mg/d, für ihre Verwendung zur Verbesserung der Schutzfunktion der Darmschleimhaut im Rahmen von Pathologien der Darmschleimhaut, ausgewählt unter den Gastroenteritiden, den ischämischen Nekrosen und den Ulzerationen der Darmschleimhaut, die Verbesserung der Schutzfunktion des Auges im Rahmen von Augenstörungen, ausgewählt unter Wasserstress, Trockenheit des Auges, Verletzungen der Hornhaut und den Keratopathien, und bei denen die Epithelialzellen betroffen sind, verabreicht werden kann.

8. Lebensmittelzusammensetzung für ihre Verwendung nach Anspruch 7 in gastroresistenter Form.

## Claims

1. Use of at least one hyaluronic acid having a molecular weight of 30,000 to 45,000 Daltons, preferably 40,000 Daltons, or one of its salts, for the manufacture of a composition intended for improving the protective function of the intestinal mucosa within the context of pathologies of the intestinal mucosa selected from the group consisting of gastroenteritis, ischemic necrosis and ulcerations of the intestinal mucosa or the improvement of the protective function of the eye within the context of ocular disorders selected from the group consisting of water stress, dryness of the eye, lesions of the cornea and keratitis and wherein the epithelial cells are involved.

2. Use of at least one hyaluronic acid having a molecular weight of 30,000 to 45,000 Daltons, preferably 40,000 Daltons, or one of its salts, for the manufacture of a medicament intended for the prevention of pathologies of the mucous membranes of the upper and lower airways selected from the group consisting of asthma, respiratory allergies, respiratory distress syndrome.

3. Use of a hyaluronic acid according to claim 1, at a concentration of 0.1 g/l to 4 g/l, preferably of 0.2 g/l to 2 g/l.

4. Use of a hyaluronic acid according to anyone of claims to 3, **characterized in that** the composition is a food composition.

5. Pharmaceutical composition, comprising at least one hyaluronic acid having a molecular weight of 30,000 to 45,000 Daltons, preferably 40,000 Daltons, or one of its salts, formulated to be administered topically at a dose comprised between 50 mg/day and 300 mg/day, preferably 100 mg/day to 200 mg/day or to be administered orally at a dose of 0.66 mg/kg/day to 4 mg/kg/day, preferably of 1.33 mg/kg/day to 2.66 mg/kg/day for use in improving the protective function of the intestinal mucosa within the context of pathologies of the intestinal mucosa selected from the group consisting of gastroenteritis, ischemic necrosis and ulcerations of the intestinal mucosa, the improvement of the protective function of the eye within the context of ocular disorders selected from the group consisting of water stress, dryness of the eye, lesions of the cornea and keratitis and wherein the epithelial cells are involved.

6. Pharmaceutical composition for use according to claim 5, in enteric-coated form.

7. Food composition, comprising at least one hyaluronic acid having a molecular weight of 30,000 to 45,000 Daltons, preferably 40,000 Daltons, or one of its salts, formulated to be administered orally at a dose comprised between 50 mg/day and 300 mg/day, preferably 100 mg/day to 200 mg/day for use in improving the protective function of the intestinal mucosa within the context of pathologies of the intestinal mucosa selected from the group consisting of gastroenteritis, ischemic necrosis and ulcerations of the intestinal mucosa, the improvement of the protective function of the eye within the context of ocular disorders selected from the group consisting of water stress, dryness of the eye, lesions of the cornea and keratitis and wherein the epithelial cells are involved.

8. Food composition for use according to claim 7, in enteric-coated form.
